# EUROPÄISCHE PATENTSCHRIFT

(11) **EP 1 942 864 B1**
(45) Veröffentlichungstag und Bekanntmachung des Hinweises auf die Patenterteilung: **14.09.2016**
(21) Anmeldenummer: 06807431.9
(22) Anmeldetag: 20.10.2006
(51) Int. Cl.: A61K 8/34, A61Q 5/02, A61Q 19/00, A61Q 19/10, A61K 8/04, A61K 8/06

(54) **KOSMETISCHE ZUBEREITUNG IN FORM EINER O/W-EMULSION ENTHALTEND 1,2-ALKANDIOL(E)**
COSMETIC PREPARATION IN THE FORM OF AN OIL-IN-WATER EMULSION CONTAINING 1,2-ALKANEDIOL(S)
PRÉPARATION COSMÉTIQUE SE PRÉSENTANT SOUS LA FORME D'UNE ÉMULSION HUILE DANS EAU CONTENANT DE L'ALCANEDIOL 1,2

(30) Priorität: 25.10.2005 DE 102005051865
(43) Veröffentlichungstag der Anmeldung: 16.07.2008
(73) Patentinhaber: Beiersdorf AG, 20253 Hamburg (DE)
(72) Erfinder: NIELSEN, Jens, 24558 Henstedt-Ulzburg (DE); BLECKMANN, Andreas, 22926 Ahrensburg (DE); ARGEMBEAUX, Horst, 21465 Wentorf (DE); DETERT, Marion, 22455 Hamburg (DE); RUPPERT, Stephan, 20259 Hamburg (DE); ALBRECHT, Harald, CH-8320 Fehraltorf (CH); KÜTHER, Jörg, 25469 Halstenbek (DE); AECHTNER, Anja, 68165 Mannheim (DE); THOMPSON, Susan, Chelmsford CM1 2NF (GB); KÖHLER, Manuela, 22147 Hamburg (DE)
(86) Internationale Anmeldenummer: PCT/EP2006/067614
(87) Internationale Veröffentlichungsnummer: WO 2007/048757

(56) Entgegenhaltungen:
- EP-A- 1 426 029
- WO-A-03/069994
- WO-A-2004/050045
- DE-A1- 10 318 526
- DATABASE WPI Week 200464 Derwent Publications Ltd., London, GB; AN 2004-656547 XP002413331 "Cosmetic for Cleaning" & JP 2004 250332 A (POLA CHEM IND INC) 9. September 2004 (2004-09-09)

## Beschreibung

Die vorliegende Erfindung betrifft ölhaltige und tensidhaltige Reinigungszubereitungen mit verbessertem Schaumverhalten, insbesondere Duschprodukte.

Der Wunsch nach einem sauberen und gepflegten Äußeren ist wohl so alt wie die Menschheit. Unreine Haut und ein ungepflegtes Haarkostüm bieten idealen Nährboden und Heimstatt für Krankheitserreger und Parasiten aller Art. Die Lust an der Körperhygiene wurde stetig verstärkt, als in den 60er Jahren des 20. Jahrhunderts neben der "klassischen" Seife auch flüssige Reinigungsmittel mit neuentwickelten synthetischen Tensiden formuliert werden konnten. Baden und Duschen sind seitdem aus unserem täglichen Leben nicht mehr wegzudenken und den Verbrauchern stehen heutzutage eine Vielzahl von Produkten für die Reinigung der verschiedenen Körperpartien zur Verfügung.

Kosmetische und/oder dermatologische Reinigungspräparate werden in aller Regel in Form eines Schaums mit Wasser auf die zu reinigenden Körperpartien aufgetragen. Basis fast aller kosmetischen oder dermatologischen Reinigungspräparate sind waschaktive Tenside. Tenside sind amphiphile Stoffe, die organische, unpolare Substanzen in Wasser lösen können. Sie zeichnen sich durch ein ambivalentes Verhalten gegenüber Wasser und Lipiden aus: Das Tensidmolekül enthält mindestens je eine hydrophile und eine lipophile Gruppe, die die Anlagerung an der Grenzfläche zwischen diesen beiden Substanzklassen ermöglichen. Auf diese Weise sorgen Tenside für eine Herabsetzung der Oberflächenspannung des Wassers, die Benetzung der Haut, die Erleichterung der Schmutzentfernung und -lösung, ein leichtes Abspülen und -je nach Wunsch - auch für Schaumregulierung. Damit ist die Grundlage für die Schmutzentfernung lipidhaltiger Verschmutzungen gegeben.

Die hohe Reinigungsleistung tensidhaltiger Reinigungszubereitungen insbesondere gegenüber Lipiden, birgt jedoch auch eine Reihe von dermatologischen Nachteilen in sich: Bereits bei einer Reinigung der Haut mit Hilfe von Wasser - ohne Zusatz von Tensiden - kommt es zunächst zu einer Quellung der Hornschicht der Haut. Der Grad dieser Quellung hängt u. a. von der Dauer des Bades und dessen Temperatur ab. Gleichzeitig werden wasserlösliche Stoffe ab- bzw. ausgewaschen, wie z. B. wasserlösliche Schmutzbestandteile, aber auch hauteigene Stoffe, die für das Wasserbindungsvermögen der Hornschicht verantwortlich sind (so genannte Feuchthaltmittel oder Moisturizer). Durch hauteigene oberflächenaktive Stoffe werden außerdem auch Hautfette in gewissem Ausmaß gelöst und ausgewaschen. Dies bedingt nach anfänglicher Quellung eine nachfolgende Austrocknung der Haut.

Es ist verständlich, dass waschaktive Tenside, die Haut und Haar von fettigen und wasserlöslichen Schmutzbestandteilen reinigen sollen, auch eine entfettende Wirkung auf die normalen Hautlipide haben. Bei jeder Hautreinigung werden in unterschiedlichem Maß auch interkorneozytäre Lipide und Sebumbestandteile entfernt. Das bedeutet, dass der natürliche Wasser-Lipid-Mantel der Haut bei jedem Waschvorgang mehr oder weniger gestört wird. Dies kann besonders bei extremer Entfettung zu einer kurzzeitigen Veränderung der Barrierefunktion der Haut führen, wobei selbstverständlich auch der jeweilige Zustand der behandelten Hautregion auf die dargestellten Veränderungen von erheblichem Einfluss ist. Beispielsweise kann die Hautdicke, die Anzahl der Talg- und Schweißdrüsen sowie die damit verbundene Empfindlichkeit erheblich variieren.

Grundsätzlich gilt dementsprechend als Forderung an waschaktive Tenside, dass sie biologisch möglichst inaktiv sind, um unerwünschte Nebenwirkungen zu vermeiden. Sie sollen ihre reinigende Wirkung bei optimaler Milde, bester Hautverträglichkeit und geringer Entfettung entfalten.

Es hat daneben aber auch nicht an Versuchen gefehlt, geeignete Reinigungszubereitungen zu finden, welche die Haut bei guter Reinigungsleistung gleichzeitig regenerieren bzw. "rückfetten". Allerdings bleibt die erzielte Leistung häufig hinter der erwarteten zurück, so dass der Anwender in aller Regel auf separate Pflegeprodukte zurückgreifen muss, welche nach der Reinigung auf die Haut aufgetragen werden und auf dieser verbleiben (so genannte "leave-on" Produkte).

Es sind nach dem Stande der Technik eine Reihe von Reinigungszubereitungen, beispielsweise Duschöle, bekannt, die zur gleichzeitigen Reinigung und Rückfettung der Haut eingesetzt werden können. Auch rückfettende Reinigungszubereitungen auf der Basis von Emulsionen sind dem Fachmanne bekannt. So beschreibt die EP 1166772 beispielsweise Reinigungsemulsionen mit einem hohen Ölgehalt. DE 10153023 offenbart Reinigungsemulsionen mit 1 bis 30 Gew.-% an Tensiden mit einem HLB-Wert von mehr als 15, 35 bis 50 Gew.-% Ölkomponenten, 0,001 bis 30 Gew.-% Wirkstoffe, 0,2 bis 5 Gew.-% bestimmter Polyacrylate und Wasser. DE 10161171 offenbart Reinigungsemulsionen mit 1 bis 30 Gew.-% an Tensiden mit einem HLB-Wert von mehr als 15, 35 bis 50 Gew.-% Ölkomponenten, 0,1 bis 10 Gew.-% Talkum, 0,2 bis 5 Gew.-% bestimmter Polyacrylate und Wasser. DE 10318526 offenbart eine Reinigungemulsion mit einer Viskosität von 500 bis 3500 mPa s bei 100 1/s und 2 bis 17 Gew.-% Natriumlaurethsulfat und/oder Natriummyrethsulfat , ein oder mehrere bestimmte Polyacrylate, 43 - 51 Gew.-% Ölphase, 25 bis 50 Gew.-% Paraffinöl und 1 bis 25 Gew.-% Öle mit einer Polarität von 5 bis 50 mN/m. EP 1458337 offenbart eine flüssige wässrige Zubereitung mit Aniontensid, Alkanolamid, Electrolyt und Wasser, wobei die Zubereitung nichtnewtonisches Verhalten aufweist.

Derartige Zubereitungen des Standes der Technik weisen jedoch eine Reihe von Nachteilen auf:
- Das Schaumverhalten der Zubereitungen lässt zu wünschen übrig. Die Zubereitungen schäumen in der Regel schlecht d. h. die Schaummenge ist zu gering, obwohl sie teilweise große Mengen an waschaktiven Tensiden enthalten da die enthaltenen Öle schaumbremsend wirken. Je höher der Ölgehalt der Emulsionen, umso stärker wird das Schaumverhalten negativ beeinflusst. Hierunter leidet die subjektiv vom Anwender empfundene und die objektive Reinigungsleistung der Zubereitung.
- Die Verteilbarkeit der Zubereitung auf der Haut ist unbefriedigend. Die meist zähflüssigen, hydrophoben Zubereitungen lassen sich relativ schwer auf der (mit Wasser benetzten) Haut verteilen.
- Die auf die Haut aufgetragene Zubereitung lässt sich relativ schwer wieder abspülen. Die Ursache hierfür sind ebenfalls die rheologischen Eigenschaften der Zubereitungen und ihre Polarität.
- Damit die Reinigungszubereitungen auf Emulsionsbasis stabil sind, müssen den Formulierungen große Mengen an Verdickern, beispielsweise Polyacrylate, zugesetzt werden. Größere Mengen an Polyacrylaten verschlechtern jedoch die rheologischen Eigenschaften der Zubereitungen. Die Viskosität wird erhöht, wodurch sich die Zubereitungen schwerer auf der Haut verteilen und nach der Anwendung wieder abspülen lassen. Auch schäumen die Zubereitungen kaum noch. Die Ursache hierfür ist vermutlich die Tatsache, dass die Mischbarkeit mit Wasser bei zunehmenden Gehalt an Polyacrylaten abnimmt. Bei Polyacrylat-Konzentrationen von unter 0,75 Gew.-% der Zubereitung hingegen, ließen sich bisher keine stabilen Reinigungsemulsionen formulieren.
- Die rückfettende Wirkung ist mangelhaft.
- Die Reinigungszubereitungen entfernen gleichzeitig und relativ unselektiv den (lipophilen) Schmutz auf der Haut ebenso wie die hauteigenen Lipide.

Es war daher die Aufgabe der vorliegenden Erfindung, die Mängel des Standes der Technik zu beseitigen oder zumindest zu lindern und eine Reinigungsemulsion zu entwickeln, deren Schaumqualität und Menge deutlich erhöht, deren Verteilbarkeit, Abspülverhalten und Stabilität deutlich verbessert, deren rückfettende Wirkung verstärkt und deren Selektivität bei der Entfernung lipophiler Bestandteile erhöht ist.

Überraschend wurde gefunden, dass der Zusatz von 1,2-Alkandiolen zu den tensid- und stark ölhaltigen Reinigungsprodukten insbesondere das Schaumvermögen der Zubereitung erheblich verbessert.

Gegenstand der Erfindung ist daher eine kosmetische Zubereitung in Form einer Öl-in-Wasser-Emulsion zur Reinigung von Haut und Haar enthaltend
eine Lipidphase,
ein oder mehrere waschaktive Tenside, die einen HLB-Wert von wenigstens 15 aufweisen und gewählt werden unter anionischen Sulfattensiden und
wenigstens ein 1,2-Alkandiol mit 5 oder 6 Kohlenstoffatomen,
dadurch gekennzeichnet, dass sie mindestens 10 Gew.% und höchstens 50 Gew.% Öl enthält.

Als besonders geeignet hat sich das 1,2-Hexandiol herausgestellt.

Der Einsatz von 1,2-Hexandiol in kosmetischen Emulsionen ist dem Fachmann an sich bekannt, doch konnte der Stand der Technik nicht den Weg zur vorliegenden Erfindung weisen.

Die WO 95/01151 offenbart die Verwendung von Alkandiolen mit 5 - 7 Kohlenstoffatomen in kosmetischen Zubereitungen. Erfindungsgemäße Zubereitungen mit 1,2-Hexandiol sind jedoch nicht explizit offenbart.

Die DE 103 41 179 offenbart Deodorantzusammensetzungen mit einer Kombination aus Alkan-1,2-diolen, u. a. 1,2-Octandiol und α- und/oder β-Hydroxysäuren. Erfindungsgemäße Zubereitungen mit 1,2-Hexandiol sind jedoch nicht offenbart.

EP 1078638 offenbart Lichtschutzzubereitungen mit hohen Konzentrationen an 1,2-Hexandiol.

All diesen Schriften fehlt es an Hinweisen, das 1,2-Hexandiol hilfreich verwendet werden kann, um die Schaumeigenschaften von kosmetischen, tensid- und ölhaltigen Zubereitungen zu verbessern.

Darüber hinaus kennt der Fachmann die WO 2004/050045, EP 1426029, WO 03/069994 sowie den Datenbank-Eintrag Database WPI week200464 Derwent Publications Ltd., London, GB; AN656547 XP002413331 "Cosmetic for Cleansing" & JP 2004 250332 (Pola Chem Ind Inc) 9.September 2004, die ebenfalls nicht den Weg zur vorliegenden Erfindun weisen konnten.

Es ergab sich für den Fachmann also überraschend und nicht vorhersehbar, das eine kosmetische Zubereitung in Form einer Öl-in-Wasser-Emulsion zur Reinigung von Haut und Haar enthaltend eine Lipidphase, ein oder mehrere waschaktive Tenside, die einen HLB-Wert, von wenigstens 15 aufweisen und gewählt werden unter anionischen Sulfattensiden und wenigstens ein 1,2-Alkandiol mit 5 oder 6 Kohlenstoffatomen, dadurch gekennzeichnet, dass sie mindestens 10 Gew.% und höchstens 50 Gew.% Öl enthält, den Mängeln des Standes der Technik abhilft und insbesondere ein besonders ausgeprägtes Schaumvermögen der Zubereitung ermöglicht. Darüber hinaus werden besonders hohe Langzeitstabilität, gute Reinigungsleistung und gutes Hautgefühl erreicht. Insbesondere die Rückfettung der Haut nach Anwendung der Zubereitung ist besonders hoch. Die Herstellung einer langzeitstabilen tensidhaltige Emulsion mit hohem Lipidanteil war überraschend, weil Prognosen über die Langzeitstabilität solcher Systeme schwer zu geben sind und insofern die Eigenschaften dieser Formulierung erstaunlich sind. Neben den Pflege- und/ oder Reinigungseigenschaften stellt die Handhabung eines kosmetischen Produktes einen für den Verbraucher sehr wichtigen Punkt dar. Die hier beschriebene Formulierung verfügt über sehr gute Anwendungseigenschaften. Sie lässt sich leicht entnehmen und dosieren. Zudem ist sie auf der Haut gut verteilbar und lässt sich nach der Anwendung einfach abspülen.

Weiterhin ist es von großem Vorteil, wenn das Alkandiol 1,2-Hexandiol ist. Dadurch ist das Schaumvermögen außergewöhnlich hoch.

Weiterhin ist es von großem Vorteil, wenn mehr als 0,3 Gew.-%, bevorzugt mehr als 0,6 Gew.-%, besonders bevorzugt mehr als 0,8 Gew.-% Alkandiol enthalten sind.

Weiterhin ist es von großem Vorteil, wenn weniger als 3 Gew.-% Alkandiol enthalten sind.

Weiterhin ist es von großem Vorteil, wenn die Lipidphase aus Triglyceriden und/oder Mineralölen besteht und ihr Gewichtsanteil an der gesamten Zubereitung mehr als 5%, bevorzugt mehr als 10%, besonders bevorzugt mehr als 20% beträgt. Dies bewirkt eine noch bessere Rückfettung der Haut.

Weiterhin ist es von grossem Vorteil, wenn die Tenside einen HLB-Wert von wenigstens 15 aufweisen und bevorzugt gewählt werden unter anionischen Sulfattensiden, besonders bevorzugt Alkylsulfaten oder Alkylethersulfaten.Dies führt besonders in Kombination mit Alkandiolen zu einem besonders guten Schaumverhalten.

Weiterhin ist es von großem Vorteil, wenn zusätzlich ein oder mehrere Polyacrylate enthalten sind, welche bevorzugt gewählt werden aus der Gruppe, die gebildet wird aus anionischen Homo- und/oder Copolymeren der Acrylsäure und/oder alkylierten Acrylsäurederivaten sowie deren Estern, wobei diese Polyacrylate besonders bevorzugt quervernetzt sind. Dadurch wird zusätzlich eine sehr gute Langzeitstabilität erreicht.

Weiterhin ist es von großem Vorteil, wenn die erfindungsgemäße Zubereitung mehr als 5 Gew.% ungesättigter Lipide enthält. Solche Zubereitungen zeichnen sich durch eine besonders hohe Hautpflegeleistung aus.

Weiterhin ist es von großem Vorteil, wenn die erfindungsgemäße Zubereitung mindestens 10 Gew.% und höchstens Gew.50% Öl enthält.

Die erfindungsgemäße Zubereitung weist vielfache Vorteile auf:
- Durch gute Schaum- und sehr gute Reinigungsleistung des Produktes ist es dem Verbraucher möglich, das Produkt als vollständigen Ersatz seines üblicherweise verwendeten nicht rückfettenden Reinigungsgels zu verwenden. Hierdurch wird eine tägliche Anwendung ermöglicht, was durch kummulative Effekte den Rückfettenden Effekt des Produktes verstärkt und eine Wirkung auf die Geschmeidigkeit und Feuchte der Haut bewirkt.
- Sowohl objektiv nachvollziehbare als auch subjektiv spürbare Rückfettung generiert ein gepflegtes Hautgefühl. Dies ermöglicht Menschen mit normaler Haut auf zusätzliche rückfettende Körperpflegemittel, deren Einsatz nach Dusche und Bad sehr verbreitet ist, zu verzichten. Dies wird erreicht durch Wahl der Lipidphase aus Triglyceriden und/oder Mineralölen, bevorzugt so dass sie mehr als 5 Gew.-% ungesättigter Lipide enthält, oder durch Gehalt von mindestens 10 Gew.-% und höchstens Gew.50% Öl.
- Der hier gewählte Formulierungstyp einer Emulsion weist eine sehr attraktive optische Anmutung für den Verbraucher auf.

Die Erfindung umfasst auch die Verwendung von 1,2-Alkandiolen mit 5, 6, 7 oder 8 Kohlenstoffatomen zur Verbesserung des Schaumvermögens von emulsionsförmigen kosmetischen Reinigungszubereitungen.

Als die anionischen, amphoteren und/oder nichtionischen Tenside im Sinne dieser Erfindung können folgende Komponenten eingesetzt werden:

### A. Anionische Tenside

Vorteilhaft zu verwendende anionische Tenside sind
Acylaminosäuren (und deren Salze), wie
1. Acylglutamate, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoyl-hydrolysiertes Soja Protein und Natrium-/ Kalium-Cocoyl-hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,
5. Acyllactylate, Lauroyllactylat, Caproyllactylat
6. Alaninate
Carbonsäuren und Derivate, wie
1. Carbonsäuren, beispielsweise Laurinsäure, Aluminiumstearat, Magnesiumalkanolat und Zinkundecylenat,
2. Ester-Carbonsäuren, beispielsweise Calciumstearoyllactylat, Laureth-6-Citrat und Natrium PEG-4-Lauramidcarboxylat,
3. Ether-Carbonsäuren, beispielsweise Natriumlaureth-13-Carboxylat und Natrium PEG-6-Cocamide Carboxylat,
Phosphorsäureester und Salze, wie beispielsweise DEA-Oleth-10-Phosphat und Dilaureth-4 Phosphat,
Sulfonsäuren und Salze, wie
1. Acyl-isethionate, z. B. Natrium-/ Ammoniumcocoyl-isethionat,
2. Alkylarylsulfonate,
3. Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat, Dinatriumundecylenamido-MEA-Sulfosuccinat und PEG-5 Laurylcitrat Sulfosuccinat.
sowie
Schwefelsäureester, wie
1. Alkylethersulfat, beispielsweise Natrium-, Ammonium-, Magnesium-, MIPA-, TIPA-Laurethsulfat, Natriummyrethsulfat und Natrium C₁₂₋₁₃-Parethsulfat,
2. Alkylsulfate, beispielsweise Natrium-, Ammonium- und TEA-Laurylsulfat.

### B. Amphotere Tenside

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

### C. Nicht-ionische Tenside

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7. Polyglycerinester, Diglycerinester, Monoglycerinester
8. Methylglucosester, Ester von Hydroxysäuren

Vorteilhaft ist ferner die Verwendung einer Kombination von anionischen und/oder amphoteren Tensiden mit einem oder mehreren nicht-ionischen Tensiden.

Vorteilhaft können zusätzlich kationische Tenside eingesetzt werden. Insbesondere können Verbindungen aus der Gruppe der quaternären Ammoniumverbindungen, insbesondere Benzyltrialkylammoniumsalze oder Alkyltrimethylammoniumsalze, beispielsweise Cetyltrimethylammoniumchlorid oder-bromid oder-methosulfat, Alkyldimethylhydroxyethylammoniumchloride oder -bromide oder - methosulfat, Dialkyldimethylammonium-chloride oder -bromide oder - methosulfat, Esterquats wie z. B. Diacylethyl Hydroxyethylmethylammoniumchlorid oder -bromid oder -methosulfat, Alkylamidethyltrimethylammoniumethersulfate, Hydroxyethyl Cetyldimonium Phosphate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyl-dimethylaminoxide eingesetzt werden.

Erfindungsgemäß besonders bevorzugt ist es, wenn als kationisches Tensid Hydroxyethylcetyldimoniumphosphat eingesetzt wird.

Beispielsweise kann das folgende kationische Hydroxyethylcetyldimoniumphosphat eingesetzt werden: Luviquat Mono CP (Fa. BASF); INCI: Hydroxyethyl Cetyldimonium Phosphate.

Erfindungsgemäß vorteilhafte Ausführungsformen der vorliegenden Erfindung sind auch dadurch gekennzeichnet, dass die Zubereitung von 0,01 bis 0,5 Gew.-% Panthenol und/oder Niacinamid enthält, wobei sich die Gewichtsangabe auf das Gesamtgewicht der Zubereitung bezieht.

Ungesättigte Lipide im Sinne der vorliegenden Schrift sind Beispielsweise Olivenöl, Sonnenblumenöl, Sojaöl, Erdnussöl, Reisöl, Rapsöl, Mandelöl, Palmöl, Rizinusöl, Kokosöl, Palmkernöl und dergleichen mehr.

Nicht zuletzt sind erfindungsgemäß vorteilhafte Ausführungsformen der vorliegenden Erfindung dadurch gekennzeichnet, dass die Zubereitung Parabene in einer Gesamtkonzentration von bis zu 0,5 Gew.-%, bezogen auf das Gesamtgewicht der Zubereitung bezieht.

Die kosmetischen und dermatologischen Zubereitungen können erfindungsgemäß kosmetische Hilfsstoffe enthalten, wie sie üblicherweise in solchen Zubereitungen verwendet werden, z. B. Konservierungsmittel, Antioxidanien, Bakterizide, Parfüme, Emulgatoren, weich machende, anfeuchtende und/oder feuchthaltende Substanzen, Fette, Öle, Wachse, Farbstoffe, Pigmente, die eine färbende Wirkung haben, Verdickungsmittel oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate. Auch die Einarbeitung von UV-Lichtschutzfiltern ist erfindungsgemäß vorteilhaft. Die aufgeführte Liste an Zusatzstoffen soll selbstverständlich nicht limitierend sein.

Eine gegebenenfalls gewünschte Ölkomponente der kosmetischen oder dermatologischen Reinigungszubereitungen - beispielsweise in Form von Reinigungsemulsionen - im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölkomponente vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölkomponente gewählt aus der Gruppe 2-Ethylhexylisostearat, Octyldodecanol, Isotridecylisononanoat, Isoeicosan, 2-Ethylhexylcocoat, C12-15-Alkylbenzoat, Capryl-Caprinsäure-triglycerid, Dicaprylylether.

Besonders vorteilhaft sind Mischungen aus C12-15-Alkylbenzoat und 2-Ethylhexylisostearat, Mischungen aus C12-15-Alkylbenzoat und Isotridecylisononanoat sowie Mischungen aus C12-15-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölkomponente ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die Ölkomponente wird ferner vorteilhaft aus der Gruppe der Phospholipide gewählt. Die Phospholipide sind Phosphorsäureester acylierter Glycerine. Von größter Bedeutung unter den Phosphatidylcholinen sind beispielsweise die Lecithine, welche sich durch die allgemeine Struktur 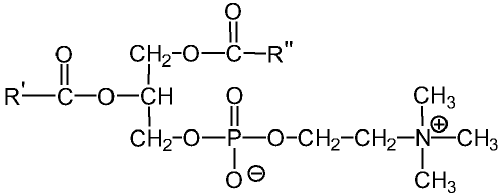 auszeichnen, wobei R' und R" typischerweise unverzweigte aliphatische Reste mit 15 oder 17 Kohlenstoffatomen und bis zu 4 cis-Doppelbindungen darstellen.

Die Zusammensetzungen enthalten gemäß der Erfindung außer den vorgenannten Substanzen gegebenenfalls die in der Kosmetik üblichen Zusatzstoffe, beispielsweise Parfüm, Farbstoffe, antimikrobielle Stoffe, rückfettende Agentien, Komplexierungs- und Sequestrierungsagentien, Perlglanzagentien, Pflanzenextrakte, Vitamine, Wirkstoffe, Konservierungsmittel, Bakterizide, Pigmente, die eine färbende Wirkung haben, Verdickungsmittel, weich machende, anfeuchtende und/oder feuchthaltende Substanzen, oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Die nachfolgenden Beispiele sollen die vorliegende Erfindung verdeutlichen ohne sie einzuschränken. Die Angaben beziehen sich stets auf Gew.-%, sofern nicht andere Angaben gemacht werden.

### Beispiele

| | **1** | **2** | **3** | **4** | **5** |
|---|---|---|---|---|---|
| Paraffinöl | 46% | 14% | 20% | 20% | 25% |
| Sojaöl | 24,3% | 36% | 20% | 20% | 25% |
| Mandelöl | - | - | - | - | - |
| Jojobaöl | - | - | - | - | - |
| Natriumlaurylethersulfat | 7,35% | 12,3% | 11% | 11% | 11% |
| Natriumbenzoat | 0,3% | 0,3% | 0,3% | - | 0,3% |
| Natriumsalicylat | 0,2% | 0,2% | 0,2% | - | 0,2% |
| Acrylates/C10-C30 Alkyl Acrylate Crosspolymer | - | - | 1% | 1% | 0,8% |
| Natriumhydroxid | - | - | 0,2% | 0,2% | 0,2% |
| Decylpolyglucoside | - | 2% | - | - | - |
| PEG-7 Glyceryl Cocoate | - | - | 1 % | 1 % | - |
| Quaternäres Ammoniumsalz der Hydroxyethylcellulose | - | - | - | - | 0,2% |
| 1,2-Hexandiol | 0,6% | 1% | 0,8% | 2% | 1,5% |
| Phenoxethanol + Methylparaben + Butylparaben + Ethylparaben + Isobutylparaben + Propylparaben | 1% | 0,7% | 1% | 1% | 0,8% |
| Butylhydroxytoluol | 0,5% | 0,5% | 0,3% | - | - |
| Parfum | q.s. | q.s. | q.s. | q.s. | q.s. |
| Wasser | ad 100 | ad 100 | ad 100 | ad 100 | ad 100 |

| | **6** | **7** | **8** | **9** | **10** |
|---|---|---|---|---|---|
| Paraffinöl | 10% | 20% | - | - | 5% |
| Sonnenblumenöl | 10% | - | 20% | 6,67% | 15% |
| Petrolatum | 2% | - | - | 3,33% | |
| Mandelöl | - | - | 1% | - | - |
| Jojobaöl | - | 1% | - | - | - |
| Natriumlaurylethersulfat | 7,5% | 11% | 11% | - | 13% |
| Natrium Tridecethsulfat | - | - | - | 14,55 % | - |
| Cocamide MIPA | - | - | - | 3% | - |
| Natriumbenzoat | 0,3% | 0,3% | 0,3% | - | 0,3% |
| Natriumsalicylat | 0,2% | 0,2% | 0,2% | - | 0,2% |
| Natrium Lauroamphoacetat | - | - | - | 4,8% | |
| Glycerin | 1% | - | - | 1% | 1% |
| Acrylates/C10-C30 Alkyl Acrylate Crosspolymer | 1% | 1,3% | 0,7%% | - | 0,8% |
| Natriumhydroxid | 0,3% | 0,2% | 0,2% | - | 0,2% |
| Decylpolyglucoside | - | 2% | - | - | - |
| PEG-7 Glyceryl Cocoate | - | 0,5% | 1% | - | - |
| Guar Hydroxypropyltrimonium Chloride | 0,3% | - | - | 0,5% | 0,2% |
| 1,2-Hexandiol | 2% | 0,8% | 1,5% | 1% | 0,6% |
| Phenoxethanol + Methylparaben + Butylparaben + Ethylparaben + Isobutylparaben + Propylparaben | 1% | 0,7% | 1% | - | 0,8% |
| Natriumchlorid | - | - | - | 3% | - |
| Zitronensäure | - | - | - | q.s. | |
| Butylhydroxytoluol | 0,5% | 0,5% | 0,3% | 0,2% | - |
| Parfum | q.s. | q.s. | q.s. | q.s. | q.s. |
| Wasser | ad 100 | ad 100 | ad 100 | ad 100 | ad 100 |

## Patentansprüche

1. Kosmetische Zubereitung in Form einer Öl-in-Wasser-Emulsion zur Reinigung von Haut und Haar enthaltend eine Lipidphase, ein oder mehrere waschaktive Tenside, die einen HLB-Wert von wenigstens 15 aufweisen und gewählt werden unter anionischen Sulfattensiden und wenigstens ein 1,2-Alkandiol mit 5 oder 6 Kohlenstoffatomen, wobei sie mindestens 10 Gew.% und höchstens 50 Gew.% Öl enthält.

2. Zubereitung nach Anspruch 1, **dadurch gekennzeichnet, dass** das Alkandiol 1,2-Hexandiol ist.

3. Zubereitung nach einem der vorhergehenden Ansprüche **dadurch gekennzeichnet, dass** mehr als 0,3 Gew.-%, bevorzugt mehr als 0,6 Gew.-%, besonders bevorzugt mehr als 0,8 Gew.-% 1,2-Alkandiol enthalten sind.

4. Zubereitung nach einem der vorhergehenden Ansprüche **dadurch gekennzeichnet, dass** weniger als 3 Gew.-% 1,2-Alkandiol enthalten sind.

5. Zubereitung nach einem der vorhergehenden Ansprüche **dadurch gekennzeichnet, dass** Lipidphase aus Triglyceriden und/oder Mineralölen besteht und ihr Gewichtsanteil an der gesamten Zubereitung mehr als 5%, bevorzugt mehr als 10%, besonders bevorzugt mehr als 20% beträgt.

6. Zubereitung nach einem der vorhergehenden Ansprüche **dadurch gekennzeichnet, dass** zusätzlich ein oder mehrere Polyacrylate enthalten sind, welche bevorzugt gewählt werden aus der Gruppe, die gebildet wird aus anionischen Homo- und/oder Copolymeren der Acrylsäure und/oder alkylierten Acrylsäurederivaten sowie deren Estern, wobei diese Polyacrylate besonders bevorzugt quervernetzt sind.

7. Zubereitung nach einem der vorangehenden Patentansprüche **dadurch gekennzeichnet, dass** sie mehr als 5 Gew.-% ungesättigter Lipide enthält.

8. Verwendung von 1,2-Alkandiolen mit 5 oder 6 Kohlenstoffatomen zur Verbesserung des Schaumvermögens einer kosmetischen Zubereitung in Form einer Öl-in-Wasser-Emulsion zur Reinigung von Haut und Haar, enthaltend eine Lipidphase, und ein oder mehrere waschaktive Tenside gewählt aus anionischen, amphoteren und/oder nichtionischen Tensiden.

## Claims

1. Cosmetic preparation in the form of an oil-in-water emulsion for the cleansing of skin and hair, comprising a lipid phase, one or more washing-active surfactants which have an HLB value of at least 15 and are selected from anionic sulphate surfactants and at least one 1,2-alkanediol having 5 or 6 carbon atoms, where it comprises at least 10% by weight and at most 50% by weight of oil.

2. Preparation according to Claim 1, **characterized in that** the alkanediol is 1,2-hexanediol.

3. Preparation according to one of the preceding claims, **characterized in that** more than 0.3% by weight, preferably more than 0.6% by weight, particularly preferably more than 0.8% by weight, of 1,2-alkanediol are present.

4. Preparation according to one of the preceding claims, **characterized in that** less than 3% by weight of 1,2-alkanediol are present.

5. Preparation according to one of the preceding claims, **characterized in that** lipid phase consists of triglycerides and/or mineral oils and its weight fraction in the overall preparation is more than 5%, preferably more than 10%, particularly preferably more than 20%.

6. Preparation according to one of the preceding claims, **characterized in that** additionally one or more polyacrylates are present which are preferably selected from the group which is formed from anionic homopolymers and/or copolymers of acrylic acid and/or alkylated acrylic acid derivatives, and esters thereof, where these polyacrylates are particularly preferably crosslinked.

7. Preparation according to one of the preceding patent claims, **characterized in that** it comprises more than 5% by weight of unsaturated lipids.

8. Use of 1,2-alkanediols having 5 or 6 carbon atoms for improving the foaming ability of a cosmetic preparation in the form of an oil-in-water emulsion for the cleansing of skin and hair, comprising a lipid phase, and one or more washing-active surfactants selected from anionic, amphoteric and/or nonionic surfactants.

## Revendications

1. Préparation cosmétique sous la forme d'une émulsion huile dans eau pour le nettoyage de la peau et des cheveux, contenant une phase lipidique, un ou plusieurs tensioactifs actifs pour le lavage, qui présentent une valeur HLB d'au moins 15 et sont choisis parmi les tensioactifs sulfates anioniques, et au moins un 1,2-alcanediol à 5 ou 6 atomes de carbone, celle-ci contenant au moins 10 % en poids et au plus 50 % en poids d'huile.

2. Préparation selon la revendication 1, **caractérisée en ce que** l'alcanediol et le 1,2-hexanediol.

3. Préparation selon l'une quelconque des revendications précédentes, **caractérisée en ce qu'**elle contient plus de 0,3 % en poids, de préférence plus de 0,6 % en poids, de manière particulièrement préférée plus de 0,8 % en poids de 1,2-alcanediol.

4. Préparation selon l'une quelconque des revendications précédentes, **caractérisée en ce qu'**elle contient moins de 3 % en poids de 1,2-alcanediol.

5. Préparation selon l'une quelconque des revendications précédentes, **caractérisée en ce que** la phase lipidique est constituée de triglycérides et/ou d'huiles minérales, et sa proportion en poids par rapport à la préparation totale est de plus de 5 %, de préférence de plus de 10 %, de manière particulièrement préférée de plus de 20 %.

6. Préparation selon l'une quelconque des revendications précédentes, **caractérisée en ce qu'**elle contient en outre un ou plusieurs polyacrylates, qui sont de préférence choisis dans le groupe formé par les homo- et/ou copolymères anioniques de l'acide acrylique et/ou les dérivés de l'acide acrylique alkylés, ainsi que leurs esters, ces polyacrylates étant de manière particulièrement préférée réticulés.

7. Préparation selon l'une quelconque des revendications précédentes, **caractérisée en ce qu'**elle contient plus de 5 % en poids de lipides insaturés.

8. Utilisation de 1,2-alcanediols à 5 ou 6 atomes de carbone pour améliorer la capacité de moussage d'une préparation cosmétique sous la forme d'une émulsion huile dans eau pour le nettoyage de la peau et des cheveux, contenant une phase lipidique, et un ou plusieurs tensioactifs actifs pour le lavage choisis parmi les tensioactifs anioniques, amphotères et/ou non ioniques.
